# EUROPÄISCHE PATENTSCHRIFT

(11) **EP 2 131 891 B1**
(45) Veröffentlichungstag und Bekanntmachung des Hinweises auf die Patenterteilung: **13.04.2016**
(21) Anmeldenummer: 08716054.5
(22) Anmeldetag: 26.02.2008
(51) Int. Cl.: A61M 1/16

(54) **VORRICHTUNG UND VERFAHREN ZUM BEFÜLLEN UND/ODER ENTLEEREN EINES DIALYSEGERÄTES**
METHOD AND DEVICE FOR FILLING AND/OR EMPTYING A DIALYSIS MACHINE
DISPOSITIF ET PROCÉDÉ POUR REMPLIR ET/OU VIDER UN APPAREIL DE DIALYSE

(30) Priorität: 26.02.2007 DE 102007009269
(43) Veröffentlichungstag der Anmeldung: 16.12.2009
(73) Patentinhaber: Fresenius Medical Care Deutschland GmbH, 61352 Bad Homburg (DE)
(72) Erfinder: WEILHÖFER, Thomas, 97464 Niederwerm (DE); ZEHNER, Georg, 97532 Üchtelhausen (DE)
(74) Vertreter: Herrmann, Uwe
(86) Internationale Anmeldenummer: PCT/EP2008/001515
(87) Internationale Veröffentlichungsnummer: WO 2008/104367

(56) Entgegenhaltungen:
- EP-A1- 1 177 801
- US-A- 4 388 184
- US-A- 5 762 782

## Beschreibung

Die vorliegende Erfindung betrifft eine Vorrichtung und ein Verfahren zum Befüllen eines Dialysegerätes mit einer Dialysierflüssigkeit und/oder zum Abführen einer Dialysierflüssigkeit aus einem Dialysegerät. Die Erfindung betrifft des weiteren die Verwendung einer Vorrichtung zum Befüllen eines Dialysegerätes mit einer Dialysierflüssigkeit und/oder zum Abführen einer Dialysierflüssigkeit aus einem Dialysegerät.

Aus dem Stand der Technik sind Hämodialysegeräte bekannt, bei denen die Dialysierflüssigkeit nicht während einer Behandlung angesetzt wird, sondern bei denen die gesamte für eine Dialysebehandlung erforderliche Menge an Dialysierflüssigkeit vor der Behandlung in einem Tank des Dialysegerätes bereitgestellt wird. Derartige Dialysegeräte werden auch als "batch-type" Dialysegeräte bezeichnet. Bei geeigneter Anordnung des Zulaufes und des Ablaufes des Tanks kann dieser nicht nur zur Bevorratung der gebrauchsfertigen Dialysierflüssigkeit, sondern auch zur Aufnahme verbrauchter Dialysierflüssigkeit herangezogen werden. Nähere Erläuterungen zu "batch-type" Dialysegeräten finden sich in dem Artikel "Batch-type dialysis maschines" in "Replacement of Renal Function by Dialysis", 5th Edition, Kluwer Academic Publishers, 2004, 369 - 371, auf den hiermit Bezug genommen wird.

Zum Ansetzen der Dialysierflüssigkeit werden bei derartigen Geräten der Anmelderin zwei zusätzliche Geräte eingesetzt, bei denen es sich zum einen um einen Wassertank für RO-Wasser (RO = reverse osmosis) und zum anderen um eine Einheit zur Herstellung der Dialysierflüssigkeit handelt. Dabei stehen die beiden Geräte derart miteinander in Verbindung, dass mittels einer Pumpe Wasser aus dem Wassertank durch eine Kammer der Einheit zur Herstellung der Dialysierflüssigkeit geführt wird, in der sich die für die Behandlung vorgesehenen Konzentrate in fester und/oder flüssiger Form befinden. Dabei wird eine bestimmte Menge RO-Wassers durch diese Kammer hindurchgespült, ohne dass es auf ein festes Mischungsverhältnis während des Einlaufvorgangs ankommt. Die auf eine Behandlung abgestimmten Mengen des oder der Konzentrate und des Wassers mischen sich ausreichend, um im Tank des Dialysegerätes eine homogene Dialysierflüssigkeit zu erhalten.

Die US 5,762,782 offenbart ein mobiles Dialysegerät, welches eine Wasseraufbereitungseinheit, eine Steuereinheit, eine Zubereitungseinheit zur Aufbereitung der Dialysierflüssigkeit, und eine Dialyseeinheit umfassend einen Dialysatkreislauf und einen extrakorporalen Blutkreislauf aufweist. Aus der EP 1 177 801 A1 ist eine Zubereitungseinheit für Dialysierflüssigkeit bekannt, bei der Wasser aus der RO-Wasserquelle in eine Mischkammer gefördert und dort mit einem pulverförmigen Konzentrat vereinigt wird. Die US 4,388,184 offenbart schließlich ein Dialysegerät, in welchem die Aufbereitungsanlage für Dialysierflüssigkeit integriert ist.

Die beiden genannten Geräte, das heißt zum einen der RO-Wassertank und zum anderen die Einheit zur Herstellung der Dialysierflüssigkeit haben einen festen Standort in einer Dialyseklinik, d. h sie sind ortsfest angeordnet. Zu diesem Standort werden die einzelnen Dialysegeräte bewegt, damit sie dort vor einer Behandlung mit der Dialysierflüssigkeit gefüllt werden können. Die auf diese Weise mit Dialysierflüssigkeit befüllten Dialysegeräte werden sodann zum Ort der Dialysebehandlung verfahren. Im Anschluß an die Dialysebehandlung werden die Dialysegeräte an dem genannten festen Standort entleert und gereinigt bzw. desinfiziert.

Ein derartiges System zum Befüllen und Entleeren von Dialysegeräten ist mit einem vergleichsweise hohen Installationsaufwand verbunden, so dass sich dessen Anschaffung insbesondere für kleine Dialysezentren nicht lohnen kann. Abgesehen davon erweist sich das bekannte System insofern als unflexibel, als dass die Geräte, die zur Herstellung der Dialysierflüssigkeit benötigt werden, ortsfest angeordnet sind.

Es ist daher die Aufgabe der vorliegenden Erfindung, eine Vorrichtung und ein Verfahren der eingangs genannten Art dahingehend weiterzubilden, dass diese mit einem vergleichsweise geringem Aufwand bereitgestellt bzw. durchgeführt werden können und darüber hinaus gegenüber dem vorbekannten System flexibler einsetzbar sind.

Diese Aufgabe wird durch eine Vorrichtung mit den Merkmalen des Anspruchs 1 sowie durch ein Verfahren mit den Merkmalen des Anspruchs 9 gelöst.

Gemäß Anspruch 1 ist vorgesehen, dass die Vorrichtung zum Befüllen eines Dialysegerätes mit einer Dialysierflüssigkeit und/oder zum Abführen von Dialysierflüssigkeit aus einem Dialysegerät mobil ist und wenigstens einen Flüssigkeitsbehälter zur Aufnahme einer Flüssigkeit, vorzugsweise von RO-Wasser, wenigstens eine Konzentrat-Aufnahme zur Aufnahme von Konzentrat und/oder einen KonzentratAnschluß zum Anschließen eines Konzentrat enthaltenden Behältnisses sowie wenigstens eine Zubereitungseinheit aufweist, die derart angeordnet und ausgeführt ist, dass in der Zubereitungseinheit Flüssigkeit aus dem Flüssigkeitsbehälter mit Konzentrat aus der Konzentrat-Aufnahme und/oder aus dem Konzentrat enthaltenden Behältnis mischbar ist.

Durch die vorliegende Erfindung wird somit eine mobile Einheit bzw. ein mobiles Gehäuse bereitgestellt, die/das die Funktionen der oben genannten aus dem Stand der Technik bekannten Geräte zur Herstellung einer Dialysierflüssigkeit vereint. Aufgrund der Mobilität der erfindungsgemäßen Vorrichtung ist es möglich, diese zu einem Dialysegerät zu bewegen und mittels der Vorrichtung je nach Bedarf das Dialysegerät mit gebrauchsfertiger Dialysierflüssigkeit zu befüllen oder verbrauchte Dialysierflüssigkeit aus dem Dialysegerät abzuführen und dieses gegebenenfalls zu reinigen bzw. zu desinfizieren. Die einzelnen Dialysegeräte müssen zum Zwecke des Befüllens und/oder Entleerens bzw. der Reinigung und Desinfektion nicht bewegt werden, wenngleich dies im Rahmen der vorliegenden Erfindung selbstverständlich auch nicht ausgeschlossen ist.

In einer Ausgestaltung der vorliegenden Erfindung ist vorgesehen, dass die Zubereitungseinheit und die Konzentrat-Aufnahme, in die das Konzentrat eingefüllt wird, durch ein und dasselbe Bauteil gebildet werden. Denkbar ist beispielsweise, dass das Konzentrat in fester und/oder flüssiger Form in die Konzentrat-Aufnahme eingegeben wird und die Konzentrat-Aufnahme sodann mit Flüssigkeit, vorzugsweise mit RO-Wasser durchspült wird, wodurch die Dialysierflüssigkeit hergestellt wird, die sodann an ein Dialysegerät abgegeben werden kann.

An dieser Stelle sei darauf hingewiesen, dass der Begriff "Dialysierflüssigkeit" im Rahmen der vorliegenden Erfindung nicht zwingend bedeutet, dass die Dialysierflüssigkeit gebrauchsfertig ist. Denkbar ist auch der Fall, dass durch die erfindungsgemäße Vorrichtung und das erfindungsgemäße Verfahren bestimmte Bestandteile einer Dialysierflüssigkeit bereitgestellt werden und dass beispielsweise in dem Dialysegerät selbst noch weitere Zusätze hinzugefügt werden oder dass eine vollständige Durchmischung oder Lösung der Bestandteile der Dialysierflüssigkeit erst in dem Tank des Dialysegerätes erfolgt. In bevorzugter Ausgestaltung der Erfindung ist jedoch vorgesehen, dass mittels der erfindungsgemäßen Vorrichtung und des erfindungsgemäßen Verfahrens eine gebrauchsfertige Dialysierflüssigkeit bereitgestellt wird, die dem Dialysegerät zugeführt wird und dann für die Dialysebehandlung eingesetzt werden kann.

Erfindungsgemäß ist vorgesehen, dass die Zubereitungseinheit derart angeordnet und ausgeführt ist, dass sie im Betrieb der Vorrichtung von Flüssigkeit aus dem Flüssigkeitsbehälter durchspült wird. Dabei erfolgt eine Mischung des oder der Konzentrate mit der Flüssigkeit. Diese Mischung wird in den Tank eines Dialysegerätes eingespült und liegt dort in Form einer homogenen, auf den zu behandelnden Patienten abgestimmten Lösung vor.

Die Vorrichtung weist zum Zwecke der Abgabe der Dialysierflüssigkeit in ein Dialysegerät einen ersten Anschluß auf, der mit der Zubereitungseinheit in Verbindung steht oder mit dieser verbindbar ist. Dieser erste Anschluß kann beispielsweise in Form eines Adapters ausgeführt sein, der eine zuverlässige Verbindung zu einem Dialysegerät ermöglicht.

Weiterhin kann die Vorrichtung zum Zwecke des Entleerens eines Dialysegerätes einen zweiten Anschluß zur Aufnahme oder Abfuhr verbrauchter Dialysierflüssigkeit aus einem Dialysegerät aufweisen. Möglich ist es, die verbrauchte Dialysierflüssigkeit aus dem Dialysegerät zunächst in der erfindungsgemäßen Vorrichtung aufzunehmen, um sie zu einem späteren Zeitpunkt aus der Vorrichtung abzuführen. Denkbar ist es ebenfalls, die verbrauchte Dialysierflüssigkeit über die erfindungsgemäße Vorrichtung einem Abfluß zuzuleiten, mit dem die Vorrichtung in Verbindung steht.

Des weiteren kann vorgesehen sein, dass die Vorrichtung zum Zwecke des Entleerens und/oder der Reinigung eines Dialysegerätes einen dritten Anschluß zum Zuführen eines Gases oder einer Reinigungsflüssigkeit zu dem Dialysegerät aufweist. Bei dem Gas kann es sich beispielsweise um Luft handeln, der ein Desinfektionsmittel zur Desinfektion des zu entleerenden oder des bereits entleerten Dialysegerätes zugegeben wurde. Bei der Reinigungsflüssigkeit kann es sich beispielsweise um RO-Wasser handeln. Selbstverständlich kann auch die Reinigungsflüssigkeit ein geeignetes Desinfektionsmittel enthalten.

Denkbar ist, dass der zweite Anschluß, mittels dessen verbrauchte Dialysierflüssigkeit aus dem Dialysegerät abgezogen wird, und der dritte Anschluß, mittels dessen das Gas, beispielsweise mit Desinfektionsmittel angereichte Luft oder eine Reinigungsflüssigkeit zu dem Dialysegerät zugeführt wird, in einem gemeinsamen Adapter angeordnet sind, der eine zuverlässige Verbindung mit dem Dialysegerät ermöglicht.

In weiterer Ausgestaltung der Erfindung weist die Vorrichtung eine Fördereinrichtung auf, die derart angeordnet und ausgeführt ist, dass mittels der Fördereinrichtung Flüssigkeit aus dem Flüssigkeitsbehälter in die Zubereitungseinheit förderbar ist und/oder dass mittels der Fördereinrichtung eine Mischung aus Flüssigkeit und Konzentrat aus der Zubereitungseinheit zu einem Dialysegerät förderbar ist.

Des weiteren ist denkbar, dass die Vorrichtung eine Fördereinrichtung aufweist, die derart angeordnet und ausgeführt ist, dass mittels der Fördereinrichtung Gas oder Flüssigkeit, beispielsweise mit einem Desinfektionsmittel angereicherte Luft oder Flüssigkeit bzw. eine Spüllösung, zu dem dritten Anschluß der Vorrichtung förderbar ist.

Dabei ist denkbar, dass die beiden genannten Fördereinrichtungen durch ein und dasselbe Bauteil gebildet werden. Denkbar ist beispielsweise, dass die Fördereinrichtung durch eine Pumpe oder durch einen Kompressor gebildet wird.

In weiterer Ausgestaltung der Erfindung ist vorgesehen, dass in der Leitung zwischen der Fördereinrichtung und dem dritten Anschluß ein Behältnis zur Aufnahme von Desinfektionsmittel angeordnet ist, wobei das Behältnis derart angeordnet ist, dass es an das/die die Leitung durchströmende Gas oder Flüssigkeit bzw. Desinfektionsmittel abgibt.

Die Vorrichtung kann über eine Einheit zur Herstellung von RO-Wasser verfügen. Das in dieser hergestellte RO-Wasser wird dem Flüssigkeitsbehälter zugeführt und dort bevorratet, bis es zur Herstellung der Dialysierflüssigkeit benötigt wird. Denkbar ist auch, dass die Vorrichtung einen vierten Anschluß aufweist, über den dem Flüssigkeitsbehälter eine Flüssigkeit, insbesondere RO-Wasser zuführbar ist.

Die erfindungsgemäße Vorrichtung kann somit über eine eigene Einheit zur Herstellung von RO-Wasser verfügen, zwingend erforderlich ist dies jedoch nicht. Ist eine derartige Einheit nicht vorgesehen, kann die Vorrichtung mit einem Anschluß versehen sein, über den der Vorrichtung bzw. dem Flüssigkeitsbehälter RO-Wasser aus einer Versorgungsleitung oder aus einer externen RO-Anlage zuführbar ist.

In weiterer Ausgestaltung der Erfindung ist eine Heizeinrichtung vorgesehen, mittels derer die in dem Flüssigkeitsbehälter befindliche Flüssigkeit und/oder die dem Flüssigkeitsbehälter zugeführte Flüssigkeit und/oder die aus dem Flüssigkeitsbehälter abfließende Flüssigkeit erwärmbar ist.

Auch ist es denkbar, eine Heizeinrichtung derart anzuordnen, dass mittels dieser die Zubereitungseinheit und/oder die aus der Zubereitungseinheit abfließende Mischung erwärmbar ist.

Die vorliegende Erfindung betrifft des Weiteren ein Verfahren zum Befüllen eines Dialysegerätes mit einer Dialysierflüssigkeit und/oder zum Abführen von Dialysierflüssigkeit aus einem Dialysegerät, das dadurch gekennzeichnet ist, dass zum Zwecke des Befüllens des Dialysegerätes in einem ersten Schritt die Dialysierflüssigkeit in einer mobilen Vorrichtung hergestellt wird und dass in einem zweiten Schritt die Dialysierflüssigkeit von der mobilen Vorrichtung an ein Dialysegerät abgegeben wird, und/oder dass zum Zwecke des Entleerens eines Dialysegerätes mittels einer mobilen Vorrichtung Dialysierflüssigkeit aus dem Dialysegerät abgezogen wird.

Dabei kann vorgesehen sein, dass die Dialysierflüssigkeit in der Vorrichtung aus wenigstens einer Flüssigkeit, vorzugsweise aus RO-Wasser, und aus wenigstens einem Konzentrat hergestellt wird, wobei sowohl die Flüssigkeit als auch das Konzentrat in der Vorrichtung bevorratet sind.

Des weiteren ist denkbar, dass das Konzentrat einer Zubereitungseinheit zugeführt bzw. in dieser vorgelegt wird und dass zur Herstellung der Dialysierflüssigkeit die Zubereitungseinheit sodann mit einer Flüssigkeit, vorzugsweise mit RO-Wasser durchspült wird.

In weiterer Ausgestaltung der Erfindung ist vorgesehen, dass mittels eines Kompressors Druckluft erzeugt wird, und dass die Druckluft Flüssigkeit aus einem Flüssigkeitsbehälter in eine Zubereitungseinheit fördert, in der die Flüssigkeit mit Konzentrat gemischt wird.

Des weiteren kann vorgesehen sein, dass mittels eines Kompressors Druckluft erzeugt wird, und dass die Druckluft einem Dialysegerät zugeführt wird, in dem sie verbrauchte Dialysierflüssigkeit aus dem zu entleerenden Dialysegerät verdrängt.

Denkbar ist, dass die Kompressoren durch dasselbe Bauteil gebildet werden, und dass in einem ersten Betriebsmodus, in dem ein Dialysegerät mit Dialysierflüssigkeit befüllt wird, Druckluft von dem Kompressor in den Flüssigkeitsbehälter gefördert wird, und dass in einem zweiten Betriebsmodus, in dem ein Dialysegerät von Dialysierflüssigkeit entleert wird, Druckluft von dem Kompressor zu dem Dialysegerät geführt wird.

In weiterer Ausgestaltung der Erfindung ist vorgesehen, dass mittels eines Kompressors Druckluft erzeugt wird, und dass die Druckluft zum Zwecke der Spülung eines Dialysegerätes Flüssigkeit aus einem Flüssigkeitsbehälter in das Dialysegerät fördert.

In weiterer Ausgestaltung der Erfindung ist vorgesehen, dass in dem zweiten Betriebsmodus zunächst zum Zwecke der Entleerung des Dialysegerätes dem Dialysegerät Druckluft zugeführt wird, durch die Dialysierflüssigkeit aus dem Dialysegerät verdrängt wird, und dass anschließend zum Zwecke der Spülung des Dialysegerätes einem Flüssigkeitsbehälter Druckluft zugeführt wird, mittels derer Flüssigkeit dem Dialysegerät zugeführt wird.

In weiterer Ausgestaltung der Erfindung kann vorgesehen sein, dass der Druckluft vor der Zufuhr zu dem Dialysegerät ein Desinfektionsmittel zugeführt wird. Alternativ oder zusätzlich ist ebenfalls möglich, dass die zum Spülen dienende Flüssigkeit mit einem Desinfektionsmittel versehen wird.

Bei der im Rahmen des erfindungsgemäßen Verfahrens eingesetzten Vorrichtung kann es sich um eine Vorrichtung gemäß einem der Ansprüche 1 bis 8 handeln.

Die Erfindung betrifft des weiteren die Verwendung einer Vorrichtung nach einem der Ansprüche 1 bis 8 zum Befüllen eines Dialysegerätes mit einer Dialysierflüssigkeit und/oder zum Abführen von Dialysierflüssigkeit aus einem Dialysegerät.

Weitere Einzelheiten und Vorteile der Erfindung werden anhand eines in der Zeichnung dargestellten Ausführungsbeispiels näher erläutert. Dabei zeigen:
- Figur 1:: die erfindungsgemäße Vorrichtung in einer Vorderansicht,
- Figur 2:: die erfindungsgemäße Vorrichtung in einer Seitenansicht mit einer externen RO-Anlage und
- Figur 3:: eine schematische Darstellung der Komponenten sowie der diese verbindenden Leitungen der erfindungsgemäßen Vorrichtung.

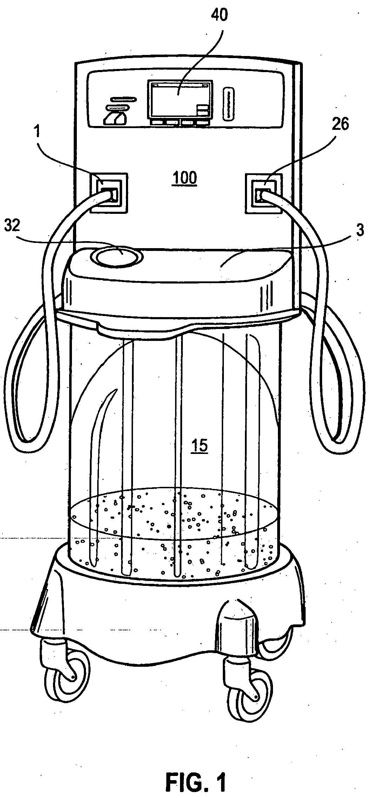

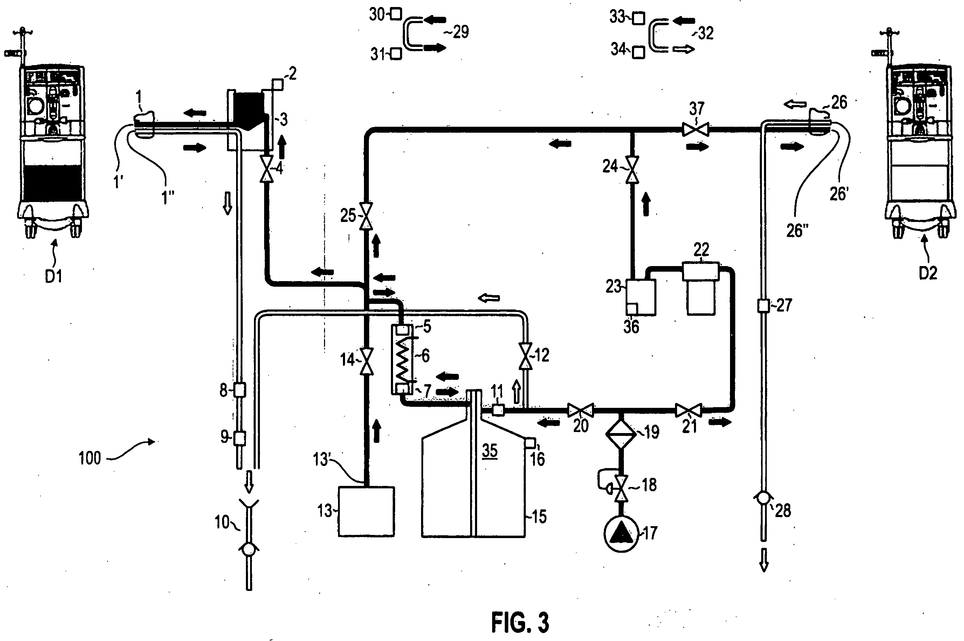

Figur 1 zeigt die erfindungsgemäße Vorrichtung 100, die zum Befüllen sowie zum Entleeren von "batch-type" Dialysegeräten D1 und D2 dient, die in Figur 3 links und rechts von der Vorrichtung 100 dargestellt sind.

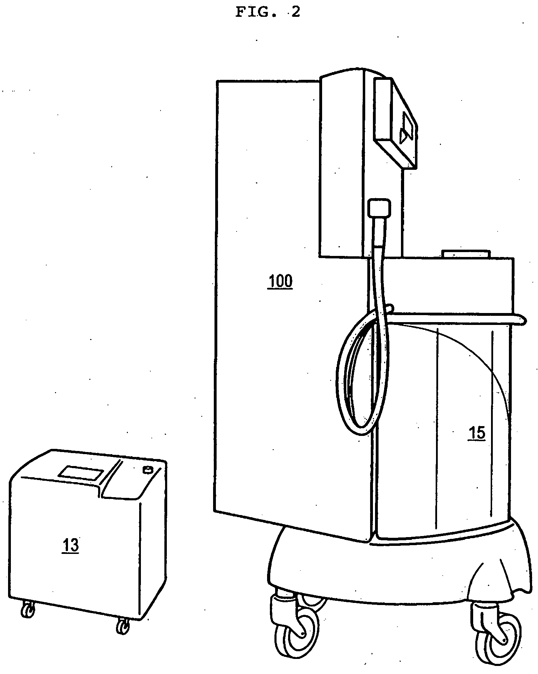

Die Vorrichtung 100 verfügt über einen Flüssigkeitsbehälter 15 sowie über eine Konzentrataufnahme 3, die zur Aufnahme des oder der Konzentrate und ferner als Zubereitungseinheit zur Zubereitung der Dialysierflüssigkeit dient. Das in Figur 1 dargestellte Gerät benötigt einen Stromanschluß, einen Abwasseranschluß und eine RO-Anlage 13, die in Figur 2 links neben der erfindungsgemäßen Vorrichtung 100 dargestellt ist. Wie bereits oben ausgeführt, ist es ebenfalls denkbar und von der Erfindung mit umfasst, nicht eine externe RO-Anlage einzusetzen, sondern eine RO-Anlage in der erfindungsgemäßen Vorrichtung 100 selbst anzuordnen.

Der Flüssigkeitsbehälter 15 wird durch einen 120 I fassenden Glastank gebildet. Oberhalb dieses Glastanks 15 befindet sich die Konzentrataufnahme und Zubereitungseinheit 3, in der das Konzentrat mit RO-Wasser aus dem Flüssigkeitsbehälter 15 gemischt wird. Die Zubereitungseinheit 3 weist eine durch einen Deckel 32 verschlossene Öffnung auf, durch die je nach Bedarf festes und/oder flüssiges Konzentrat einfüllbar ist. Die Zubereitungseinheit 3 ist als Einspültopf ausgeführt und in die Arbeitsplatte integriert. Der Deckel 32 des Einspültopfes besteht aus einer dicken Scheibe mit Griff. Nachdem dieser eingesetzt wurde, wird er durch eine Mechanik verschlossen.

Wie dies aus Figur 1 weiter hervorgeht bzw. dort angedeutet ist, sind im Frontteil der Vorrichtung 100 die notwendigen Bedienelemente für den Anwender angeordnet. Zum einen ist dort ein Touchscreen-Display 40, ein Schacht bzw. ein Kartenleser für die Gerätekarte des Dialysegerätes, sowie die Aufnahmen für die Adapter 1, 26 zum Befüllen und Entleeren sowie eine Labeldruckerausgabe angeordnet.

Für Kundendiensteinsätze kann es von Vorteil sein, wenn das Frontteil geöffnet werden kann.

Die in Figur 3 näher dargestellte Hydraulik sowie alle notwendigen Module sind unter der Arbeitsplatte sowie im hinteren Bereich des Gerätes untergebracht.

Wie bereits oben ausführt, zeigt Figur 2 die erfindungsgemäße Vorrichtung in einer Seitenansicht sowie eine externe RO-Anlage 13 als Beispiel für die Permeatversorgung. Mittels dieser RO-Anlage 13 wird RO-Wasser hergestellt und sodann in dem als Glastank ausgeführten Flüssigkeitsbehälter 15 aufgenommen und darin bevorratet, bis die Vorrichtung zur Herstellung einer Dialysierflüssigkeit und zum Füllen eines Dialysegerätes eingesetzt wird.

Werden die Adapter 1, 26 zum Befüllen bzw. zum Entleeren nicht benötigt, stecken sie wie aus Figur 1 ersichtlich in ihrer Parkposition. Dort können sie mit einer Klammer vor Herausrutschen gesichert sein. Denkbar ist, dass Sensoren vorgesehen sind, die erkennen, ob die Adapter 1, 26 gesteckt und gesichert sind. Des weiteren ist denkbar, dass eine geeignete UV-Lampe angeordnet wird, mittels derer auch an der Adapteroberfläche das Keimwachstum verhindert oder zumindest in Grenzen gehalten werden kann.

Zum Scannen von Konzentraten ist des weiteren ein Laserscanner vorgesehen, der in dem Gehäuse der Vorrichtung 100 integriert ist. Denkbar ist, dass der Scannvorgang automatisch solange aktiviert bleibt, bis die gewünschten Konzentrate erkannt wurden. Diese Anordnung eines eingebauten Scanners hat den Vorteil, dass störende Kabel entfallen und dass der Anwender die Konzentrate nur noch in den Laserstrahl zum Scannen positionieren muss.

Der Vorgang der Herstellung einer Dialysierflüssigkeit, der Abgabe der Dialysierflüssigkeit in das Dialysegerät D1 sowie des Entleerens eines Dialysegerätes D2 wird nun anhand von Figur 3 näher erläutert.

Im Rahmen der Vorbereitung der Herstellung einer Dialysierflüssigkeit wird zunächst RO-Wasser, beispielsweise aus der in Figur 2, links dargestellten RO-Anlage 13 über den Anschluß 13' bei geöffnetem Ventil 14 und bei geschlossenen Ventilen 4, 25 über einen Durchlauferhitzer 6 geführt und dabei auf 36 °C erwärmt und sodann in den Flüssigkeitsbehälter 15 der Vorrichtung 100 eingefüllt. Es wird sodann in dem Glas-Flüssigkeitsbehälter 15 bevorratet. Dieser verfügt über einen kapazitiven Sensor 16, der den Füllstand in dem Behälter 15 mißt. Des weiteren ist in dem Behälter 15 eine UV-Lampe 35 mit Schutzrohr vorgesehen, die dafür sorgt, dass der Verkeimungsprozeß nicht oder in nur untergeordnetem Ausmaße abläuft.

Ist die Verbrauchsmenge von beispielsweise 120 I erreicht, ist der Vorbereitungsprozeß beendet.

Zum Herstellen der Dialysierflüssigkeit wird/werden das oder die Konzentrate in die Zubereitungseinheit 3 eingegeben, bei der es sich um den oben genannten Einspültopf handelt, der von der erwärmten Flüssigkeit aus dem Flüssigkeitsbehälter 15 durchströmt wird.

Zunächst wird das Dialysegerät D1 mittels des Adapters 1 an der Vorrichtung 100 konnektiert und mittels der Gerätekarte an der Vorrichtung 100 angemeldet. Die Gerätekarte wird überprüft und wenn der Status in Ordnung ist, wird der Scannvorgang zum Scannen der Konzentrate freigegeben. Bei den Konzentraten handelt es sich beispielsweise um ein festes Konzentrat, das z. B. die Bestandteile NaCl, NaHCO₃ und Glucose enthalten kann, und um ein flüssiges Konzentrat, das z. B. die Komponenten KCl, CaCl₂ und MgCl₂ enthalten kann. Das oder die Konzentrate werden in den Einspültopf 3 eingegeben. Das Bezugszeichen 2 kennzeichnet einen Sensor für den Einspültopfdeckel.

Nach dem Anschließen eines Dialysegerätes D1 an die Vorrichtung 100 mittels des Adapters 1 wird der Kompressor 17 in Betrieb gesetzt und bei geöffnetem Ventil 20 und bei geschlossenen Ventilen 12 und 21 wird Druckluft in den Behälter 15 eingeführt und dadurch Flüssigkeit aus dem Behälter 15 über den Durchlauferhitzer 6 und bei geöffnetem Ventil 4 und geschlossenen Ventilen 14 und 25 in den Einspültopf 3 geführt. Dort mischt sich die temperierte Flüssigkeit mit dem oder den Konzentraten und wird über den Adapter 1 bzw. dessen Anschluß 1' dem Dialysegerät D1 zugeführt. Gleichzeitig wird über den Anschluß 1" des Adapters 1 Luft aus dem Dialysegerät D1 abgeführt. In der die Luft abführenden Leitung befindet sich eine Leitfähigkeits-/Temperaturmeßzelle 8 sowie ein Optosensor 9, über den ein etwaiger Überlauf feststellbar ist. Das Bezugszeichen 10 stellt einen Trichter mit einem Absperrventil dar.

Erfindungsgemäß ist es möglich, den Zeitpunkt insbesondere der Zugabe des flüssigen Konzentrates nicht mehr zeitgesteuert, sondern über den Füllstandssensor 16 an dem Glas-Flüssigkeitsbehälter 15 zu bestimmen. Der Füllstandsensor 16 kann somit zum einen dazu dienen, eine vorgegebene Verbrauchsmenge in den Behälter 15 einzufüllen und zum anderen dazu, den genauen Füllstand für die Zugabe eines Konzentrates zu bestimmen, wenn dies für die Auflösung und Verteilung des Konzentrates sinnvoll ist.

Sodann wird die Leitfähigkeit und die Temperatur der Dialysierflüssigkeit gemessen und ausgedruckt. Je nach Ergebnis wird die Gerätekarte zur Dialyse freigegeben, woraufhin das Dialysegerät D1 für eine Dialysebehandlung zur Verfügung steht.

Soll ein mit verbrauchter Dialysierflüssigkeit gefülltes Dialysegerät D2, wie es in Figur 3 rechts dargestellt ist, mittels der Vorrichtung 100 entleert werden, wird die Vorrichtung 100 mittels des Adapters 26 an das Dialysegerät D2 angeschlossen. Sodann wird mittels des Kompressors 17 Druckluft erzeugt und diese bei geöffnetem Ventil 21 und geschlossenem Ventil 20 in dem Desinfektionsmittelbehältnis 22 mit Desinfektionsmittel, z. B. Peressigsäure, angereichert. Die mit Desinfektionsmittel beladene Luft strömt sodann über eine Leckageüberwachung 23 mit einem Sensor 36 und bei geöffnetem Ventilen 24 und 37 sowie geschlossenem Ventil 25 über den Anschluß 26" des Adapters 26 in das Dialysegerät D2. Die Druckluft verdrängt in dem Dialysegerät D2 die darin befindliche verbrauchte Dialysierflüssigkeit. Diese gelangt über den Anschluß 26' des Adapters 26 sowie über eine Abflußleitung, die ebenfalls einen Bestandteil der Vorrichtung 100 bildet, und in der ein Optosensor 27 und ein Rückschlagventil angeordnet sind, in den Abfluß 28.

Nach dem Entleeren des Dialysegerätes D2 wird dieses mit RO-Wasser gespült wozu der Kompressor 17 Druckluft erzeugt, die bei geschlossenen Ventilen 12 und 21 und bei geöffnetem Ventil 20 in den Flüssigkeitsbehälter 15 geführt wird. Von dort aus wird Flüssigkeit nach Durchströmen des Erhitzers 6 bei geschlossenen Ventilen 4 und 24 und bei geöffneten Ventilen 25 und 37 über den Adapter 26 dem Dialysegerät D2 zugeführt und nach Durchströmen des Dialysegerätes D2 über den Abfluß 28 abgezogen.

Mit den Bezugszeichen 5 und 7 sind Temperatursensoren und Flußwächter am Ein- bzw. Ausgang des Durchlauferhitzers 6 dargestellt.

Das Bezugszeichen 12 kennzeichnet ein Ventil zur Entlüftung des Flüssigkeitsbehälters 15.

Die Bezugszeichen 18 und 19 kennzeichnen schließlich ein Überdruckventil bzw. einen Partikelfilter zur Reinigung der komprimierten Luft.

Die Bezugszeichen 29 und 32 kennzeichnen Kurzschlußstücke mit jeweils zwei Sensoren 30, 31 und 33, 34. Die Kurzschlußstücke 29, 32 werden in die Anschlüsse 1' und 1" des Adapters 1 sowie in die Anschlüsse 26' und 26" des Adapters 26 gesteckt und sind vorgesehen, damit die Adapter 1 und 26 in die aus Figur 1 ersichtliche Parkposition gesteckt werden können. Je ein Sensor erkennt den eingesteckten Adapter und je ein anderer Sensor erkennt die Sicherung des Adapters (durch eine Klammer). In dieser Position kann die gesamte Hydraulik der Vorrichtung 100 durch eine entsprechende Schaltung der Ventile automatisch mit dem Desinfektionsmittel desinfiziert sowie auch mit einer Flüssigkeit gespült werden.

## Patentansprüche

1. Vorrichtung (100) zum Befüllen eines batch-type Dialysegerätes (D1) mit einer Dialysierflüssigkeit, wobei die Vorrichtung (100) separat vom Dialysegerät ausgebildet und relativ zu diesem bewegbar ist und mobil ist und wenigstens einen Flüssigkeitsbehälter (15) zur Aufnahme einer Flüssigkeit, vorzugsweise von RO-Wasser, wenigstens eine Konzentrat-Aufnahme (3) zur Aufnahme von Konzentrat oder einen Konzentrat-Anschluss zum Anschließen eines Konzentrat enthaltenden Behältnisses, sowie wenigstens eine Zubereitungseinheit (3) aufweist, die derart angeordnet und ausgeführt ist, dass sie im Betrieb der Vorrichtung (100) von Flüssigkeit aus dem Flüssigkeitsbehälter (15) durchspült wird und dass in der Zubereitungseinheit (3) Flüssigkeit aus dem Flüssigkeitsbehälter (15) mit Konzentrat aus der Konzentrat-Aufnahme (3) oder aus dem Konzentrat enthaltenden Behältnis mischbar ist, und dass die Vorrichtung (100) zum Zwecke der Abgabe der Dialysierflüssigkeit an ein Dialysegerät (D1) einen ersten Anschluss (1') aufweist, der mit der Zubereitungseinheit (3) in Verbindung steht oder mit dieser verbindbar ist.

2. Vorrichtung (100) nach Anspruch 1, **dadurch gekennzeichnet, dass** die Zubereitungseinheit (3) und die Konzentrat-Aufnahme (3) durch dasselbe Bauteil gebildet werden.

3. Vorrichtung (100) nach einem der vorhergehenden Ansprüche, **dadurch gekennzeichnet, dass** die Vorrichtung (100) eine Fördereinrichtung (17) aufweist, die derart angeordnet und ausgeführt ist, dass mittels der Fördereinrichtung (17) im Betrieb der Vorrichtung (100) Flüssigkeit aus dem Flüssigkeitsbehälter (15) in die Zubereitungseinheit (3) und/oder eine Mischung aus Flüssigkeit und Konzentrat aus der Zubereitungseinheit (3) zu einem Dialysegerät (D1) gefördert wird.

4. Vorrichtung (100) nach Anspruch 3, **dadurch gekennzeichnet, dass** die Fördereinrichtung (17) durch eine Pumpe oder durch einen Kompressor gebildet wird.

5. Vorrichtung (100) nach einem der vorherigen Ansprüche, **dadurch gekennzeichnet, dass** die Vorrichtung (100) eine Einheit zur Herstellung von RO-Wasser aufweist.

6. Vorrichtung (100) nach einem der Ansprüche 1 bis 4, **dadurch gekennzeichnet, dass** die Vorrichtung (100) einen Anschluss (13') aufweist, über den dem Flüssigkeitsbehälter (15) eine Flüssigkeit, insbesondere RO-Wasser zuführbar ist.

7. Vorrichtung (100) nach einem der vorhergehenden Ansprüche, **dadurch gekennzeichnet, dass** eine Heizeinrichtung (6) vorgesehen ist, mittels derer die in dem Flüssigkeitsbehälter (15) befindliche Flüssigkeit und/oder die dem Flüssigkeitsbehälter (15) zugeführte Flüssigkeit und/oder die aus dem Flüssigkeitsbehälter (15) abfließende Flüssigkeit erwärmbar ist.

8. Vorrichtung (100) nach einem der vorhergehenden Ansprüche, **dadurch gekennzeichnet, dass** eine Heizeinrichtung vorgesehen ist, mittels derer die Zubereitungseinheit (3) und/oder die aus der Zubereitungseinheit (3) abfließende Mischung erwärmbar ist.

9. Verfahren zum Befüllen eines batch-type Dialysegerätes (D1) mit einer Dialysierflüssigkeit, wobei zum Zwecke des Befüllens des Dialysegerätes (D1) in einem ersten Schritt die Dialysierflüssigkeit in einer separat vom Dialysegerät ausgebildeten und relativ zu diesem bewegbaren und mobilen Vorrichtung (100) hergestellt wird, wobei in einem zweiten Schritt die Dialysierflüssigkeit von der mobilen Vorrichtung (100) an das Dialysegerät (D1) abgegeben wird, und wobei die Dialysierflüssigkeit in der Vorrichtung (100) aus wenigstens einer Flüssigkeit, vorzugsweise aus RO-Wasser, und wenigstens einem Konzentrat hergestellt wird, wobei sowohl die Flüssigkeit als auch das Konzentrat in der mobilen Vorrichtung (100) bevorratet sind.

10. Verfahren nach Anspruch 9, **dadurch gekennzeichnet, dass** das Konzentrat in einer Zubereitungseinheit (3) vorgelegt wird, dass zur Herstellung der Dialysierflüssigkeit die Zubereitungseinheit (3) mit einer Flüssigkeit durchspült wird und dass die auf diese Weise hergestellte Dialysierflüssigkeit sodann einem Dialysegerät (D1) zugeführt wird.

11. Verfahren nach Anspruch 9 oder 10, **dadurch gekennzeichnet, dass** mittels eines Kompressors (17) Druckluft erzeugt wird, und dass die Druckluft Flüssigkeit aus einem Flüssigkeitsbehälter (15) in eine Zubereitungseinheit (3) fördert, in der die Flüssigkeit mit Konzentrat gemischt wird.

12. Verwendung einer Vorrichtung nach einem der Ansprüche 1 bis 8 zum Befüllen eines batch-type Dialysegerätes (D1) mit einer Dialysierflüssigkeit.

## Claims

1. An apparatus (100) for the filling of a batch-type dialysis machine (D1) with a dialysis liquid, wherein the apparatus (100) is formed separately from the dialysis machine und movable relative to said dialysis machine and is mobile and has at least one liquid container (15) for the reception of a liquid, preferably of RO water, at least one concentrate receptacle (3) for the reception of concentrate or a concentrate connection for the connection of a container holding concentrate as well as at least one preparation unit (3) which is arranged and configured such that it is flushed by liquid from the liquid container (15) in the operation of the apparatus (100) and that liquid from the liquid container (15) can be mixed with concentrate from the concentrate receptacle (3) or from the container holding the concentrate in the preparation unit (3) and that the apparatus (100) has a first connector (1') for the purpose of the dispensing of the dialysis liquid to a dialysis machine (D1), said connector being in communication with or connectable to the preparation unit (3).

2. An apparatus (100) in accordance with claim 1, wherein the preparation unit (3) and the concentrate receptacle (3) are formed by the same component.

3. An apparatus (100) in accordance with one of the preceding claims, wherein the apparatus (100) has a conveying device (17) which is arranged and configured such that liquid is conveyed from the liquid container (15) into the preparation unit (3) by means of the conveying device (17) in the operation of the apparatus (100) and/or a mixture of liquid and concentrate is conveyed from the preparation unit (3) to a dialysis machine (D1).

4. An apparatus (100) in accordance with claim 3, wherein the conveying device (17) is formed by a pump or by a compressor.

5. An apparatus (100) in accordance with one of the preceding claims, wherein the apparatus (100) has a unit for the manufacture of RO water.

6. An apparatus (100) in accordance with one of the claims 1 to 4, wherein the apparatus (100) has connector (13') via which a liquid, in particular RO water, can be supplied to the liquid container (15).

7. An apparatus (100) in accordance with one of the preceding claims, wherein a heating device (6) is provided by means of which the liquid located in the liquid container (15) and/or the liquid supplied to the liquid container (15) and/or the liquid flowing out of the liquid container (15) can be heated.

8. An apparatus (100) in accordance with one of the preceding claims, wherein a heating device is provided by means of which the preparation unit (3) and/or the mixture flowing out of the preparation unit (3) can be heated.

9. A method for the filling of a batch-type dialysis machine (D1) with a dialysis liquid, wherein, for the purpose of filling the dialysis machine (D1), in a first step, the dialysis liquid is manufactured in a mobile apparatus (100) formed separately from the dialysis machine und movable relative to said dialysis machine, wherein, in a second step, the dialysis liquid is dispensed by the mobile apparatus (100) to the dialysis machine (D1), and wherein the dialysis liquid is manufactured in the apparatus (100) from at least one liquid, preferably from RO water, and at least one concentrate, with both the liquid and the concentrate being stored in the mobile apparatus (100).

10. A method in accordance with claim 9, wherein the concentrate is presented in a preparation unit (3), wherein the preparation unit (3) is flushed with a liquid for the manufacture of the dialysis liquid and wherein the dialysis liquid manufactured in this manner is then supplied to a dialysis machine (D1).

11. A method in accordance with claim 9 or claim 10, wherein compressed air is generated by means of a compressor (17) and wherein the compressed air conveys liquid out of a liquid container (15) into a preparation unit (3) in which the liquid is mixed with a concentrate.

12. Use of an apparatus in accordance with one of the claims 1 to 8 for the filling of a batch-type dialysis machine (D1) with a dialysis liquid.

## Revendications

1. Dispositif (100) pour remplir un appareil de dialyse (D1) de type « batch » avec un fluide de dialyse, le dispositif (100) étant conçu séparément de l'appareil de dialyse et déplaçable par rapport à celui-ci et étant mobile et comportant au moins un réservoir à fluide (15) destiné à recevoir un fluide, de préférence de l'eau traitée par osmose inverse, au moins un compartiment pour concentré (3) destiné à recevoir du concentré ou un raccord de concentré destiné à raccorder un récipient contenant du concentré, ainsi qu'au moins une unité de préparation (3), qui est disposée et conçue de manière à être parcourue par du fluide provenant du réservoir à fluide (15) pendant le fonctionnement du dispositif (100) et que du fluide provenant du réservoir à fluide (15) puisse être mélangé dans l'unité de préparation (3) avec du concentré provenant du compartiment pour concentré (3) ou du récipient contenant du concentré, et que le dispositif (100) comporte, pour distribuer le fluide de dialyse vers un appareil de dialyse (D1), un premier raccord (1'), qui est en liaison avec l'unité de préparation (3) ou peut être relié à celle-ci.

2. Dispositif (100) selon la revendication 1, **caractérisé en ce que** l'unité de préparation (3) et le compartiment pour concentré (3) sont formés par le même élément.

3. Dispositif (100) selon l'une des revendications précédentes, **caractérisé en ce que** le dispositif (100) comporte un dispositif de transport (17) qui est disposé et conçu de telle manière que du fluide est transporté du réservoir à fluide (15) dans l'unité de préparation (3) et/ou un mélange de fluide et de concentré est transporté de l'unité de préparation (3) à un appareil de dialyse (D1) au moyen du dispositif de transport (17).

4. Dispositif selon la revendication 3, **caractérisé en ce que** le dispositif de transport (17) est formé par une pompe ou par un compresseur.

5. Dispositif (100) selon l'une des revendications précédentes, **caractérisé en ce que** le dispositif (100) comporte une unité destinée à fabriquer de l'eau traitée par osmose inverse.

6. Dispositif (100) selon l'une des revendications 1 à 4, **caractérisé en ce que** le dispositif (100) comporte un raccord (13') par le biais duquel un fluide, en particulier de l'eau traitée par osmose inverse, peut être acheminé dans le réservoir à fluide (15).

7. Dispositif (100) selon l'une des revendications précédentes, **caractérisé en ce qu'**un dispositif de chauffage (6) est prévu, au moyen duquel le fluide présent dans le réservoir à fluide (15) et/ou le fluide acheminé dans le réservoir à fluide (15) et/ou le fluide s'écoulant hors du réservoir à fluide (15) peut être chauffé.

8. Dispositif (100) selon l'une des revendications précédentes, **caractérisé en ce qu'**un dispositif de chauffage est prévu, au moyen duquel l'unité de préparation (3) et/ou le mélange s'écoulant hors de l'unité de préparation (3) peut être chauffé(e).

9. Procédé pour remplir un appareil de dialyse (D1) de type « batch » avec un fluide de dialyse, dans lequel, pour remplir l'appareil de dialyse (D1), dans une première étape, le fluide de dialyse est produit dans un dispositif (100) conçu séparément de l'appareil de dialyse, déplaçable par rapport à celui-ci et mobile, dans une deuxième étape, le fluide de dialyse est distribué du dispositif (100) mobile à l'appareil de dialyse (D1), et le fluide de dialyse est produit dans le dispositif (100) à partir d'au moins un fluide, de préférence de l'eau traitée par osmose inverse, et d'au moins un concentré, aussi bien le fluide que le concentré étant stockés dans le dispositif (100) mobile.

10. Procédé selon la revendication 9, **caractérisé en ce que** le concentré est placé dans une unité de préparation (3), **en ce que** l'unité de préparation (3) est parcourue par un fluide pour produire le fluide de dialyse et **en ce que** le fluide de dialyse produit de cette manière est ensuite acheminé dans un appareil de dialyse (D1).

11. Procédé selon la revendication 9 ou 10, **caractérisé en ce que** de l'air comprimé est généré au moyen d'un compresseur (17), et **en ce que** l'air comprimé transporte du fluide d'un réservoir à fluide (15) à une unité de préparation (3), dans laquelle le fluide est mélangé avec du concentré.

12. Utilisation d'un dispositif selon l'une des revendications 1 à 8, pour remplir un appareil de dialyse (D1) de type « batch » avec un fluide de dialyse.
